# EUROPÄISCHE PATENTSCHRIFT

(11) **EP 3 220 725 B1**
(45) Veröffentlichungstag und Bekanntmachung des Hinweises auf die Patenterteilung: **22.05.2019**
(21) Anmeldenummer: 17161059.5
(22) Anmeldetag: 15.03.2017
(51) Int. Cl.: H05H 1/24, A61L 2/14, A61L 9/22

(54) **LUFTREINIGUNGSMODUL MIT EINER PLASMAERZEUGUNGSEINRICHTUNG UND VERFAHREN ZUR HERSTELLUNG EINER PLASMAERZEUGUNGSEINRICHTUNG**
AIR PURIFICATION MODULE WITH A PLASMA-GENERATING DEVICE AND METHOD FOR THE PREPARATION OF A PLASMA-GENERATING DEVICE
MODULE DE PURIFICATION D'AIR COMPRENANT UN DISPOSITIF DE PRODUCTION DE PLASMA ET PROCÉDÉ DE FABRICATION D'UN DISPOSITIF DE PRODUCTION DE PLASMA

(30) Priorität: 15.03.2016 DE 102016104805
(43) Veröffentlichungstag der Anmeldung: 20.09.2017
(73) Patentinhaber: Langner, Manfred H., 49509 Recke (DE)
(72) Erfinder: Langner, Manfred H., 49509 Recke (DE)
(74) Vertreter: Habermann Intellectual Property Partnerschaft von Patentanwälten mbB

(56) Entgegenhaltungen:
- WO-A1-2016/016273
- JP-A- 2006 236 697
- JP-A- 2008 130 343

## Beschreibung

Die Erfindung betrifft ein Luftreinigungsmodul mit einem Kamingehäuse zur Montage in einem Strömungskanal, wobei das Kamingehäuse eine erste Stirnseite, eine gegenüberliegende zweite Stirnseite und eine die beiden Stirnseiten verbindende Mantelfläche aufweist und wobei an der ersten Stirnseite mindestens eine Lufteintrittsöffnung angeordnet ist, und mit einer Plasmaerzeugungseinrichtung zur Erzeugung eines nichtthermischen Plasmas, die an einer Trägerplatte angeordnet ist, die an der zweiten Stirnseite an dem Kamingehäuse so festgelegt ist, dass die Plasmaerzeugungseinrichtung in einem von dem Kamingehäuse umgebenen Kamininnenraum angeordnet ist, wobei die Plasmaerzeugungseinrichtung mindestens zwei Plasmaelektroden aufweist, die jeweils durch mindestens einen flexiblen drahtförmigen Elektrodenleiter gebildet werden, wobei die Elektrodenleiter von den zwei Plasmaelektroden entlang mindestens eines Plasmaerzeugungsabschnitts miteinander verdrillt oder parallel nebeneinander angeordnet und nur durch ein Isolierungsmaterial voneinander beabstandet sind, so dass über die gesamte Länge des Plasmaerzeugungsabschnitts ein nichtthermisches Plasma erzeugt werden kann.

Aus der Praxis sind verschiedene Luftreinigungsmodule mit einer Plasmaerzeugungseinrichtung bekannt, die in einem Luftkanal oder in einem Strömungsbereich einer Luftströmung angeordnet werden können, um die durch das Luftreinigungsmodul hindurch strömende oder daran vorbei strömende Luft zu reinigen. Es hat sich gezeigt, dass mit einer Plasmaerzeugungseinrichtung, mit der ein nichtthermisches Plasma, beispielsweise durch eine erzwungene dielektrische Entladung, erzeugt wird, in der Luftströmung enthaltene Teilchen und Moleküle in einzelne Bestandteile, bzw. reaktive Komponenten zerlegt werden können. Durch ein geeignet erzeugtes nichtthermische Plasma können beispielsweise Geruchsstoffe oder flüchtige organische Verbindungen, welche die Gesundheit oder das Wohlbefinden beeinträchtigen, umgewandelt und dadurch aus der Luftströmung entfernt werden. Aus der Praxis sind verschiedene Ausgestaltungen derartiger Luftreinigungsmodule mit einer Plasmaerzeugungseinrichtung bekannt, die beispielsweise in Dunstabzugshauben, in industriell genutzten Ablufteinrichtungen oder in Klimageräten eingesetzt werden können.

In WO 2014/049128 A1 ist ein Luftreinigungsmodul mit einer in einem Kamingehäuse angeordneten Plasmaerzeugungseinrichtung beschrieben. Das als autarkes Standgerät vorgesehene Luftreinigungsmodul ist nicht ohne weiteres für den Einbau in einem Abluftkamin geeignet.

Aus JP H03 150206 A ist ein Luftreinigungsmodul mit einer Plasmaerzeugungseinrichtung bekannt, bei der einzelne drahtförmige Elektrodenleiter gitterartig miteinander verwoben sind und an den jeweiligen Kreuzungspunkten durch Anlegung einer geeigneten Wechselspannung ein nichtthermisches Plasma erzeugen können. Die drahtförmigen Elektrodenleiter sind in einem rechteckförmigen Rahmengestell verspannt, das in einem Abluftkanal so angeordnet werden kann, dass die Luftströmung in dem Abluftkanal durch das Rahmengestell und damit die von dem Elektrodengitter aufgespannte Fläche durchströmen kann.

Beispielsweise aus WO 2016/016273 ist ein Luftreinigungsmodul bekannt, bei dem verdrillte oder parallel geführte und eng aneinander anliegende drahtförmige Elektrodenleiter Plasmaelektroden bilden. Die verdrillten oder parallel geführten drahtförmigen Elektrodenleiter werden zwischen den Wangen eines rechteckigen Rahmengestells verspannt oder auf einer in dem Rahmengestell angeordneten Filtermatte festgelegt, so dass ein durch das Rahmengestell hindurch strömender Luftstrom zwischen den einzelnen Plasmaerzeugungsabschnitten der Elektrodenleiter hindurch strömen kann.

Die vorteilhaften Reinigungseigenschaften und insbesondere die Möglichkeit, mit einem geringen Energieverbrauch Geruchsstoffe und flüchtige organische Verbindungen aus einem Abluftstrom entfernen zu können, legen eine Verwendung derartiger Luftreinigungsmodule in Dunstabzugshauben oder in Klimatisierungseinrichtungen nahe. Insbesondere bei Dunstabzugshauben oder Klimatisierungsgeräten, die in großer Stückzahl und kostengünstig hergestellt werden und sich an Endverbraucher richten, bzw. zur Verwendung in Wohngebäuden vorgesehen sind, sollten die Herstellungs- und Montagekosten eines derartigen Luftreinigungsmoduls möglichst gering sein. Die Verwendung der flexiblen drahtförmigen Elektrodenleiter ermöglicht zwar eine raumsparende Ausgestaltung der Plasmaelektroden und deren Anordnung innerhalb von Strömungskanälen in einer Dunstabzugshaube oder in einem Klimatisierungsgerät, erfordert jedoch eine aufwendige und in aller Regel manuelle Verlegung und Festlegung der drahtförmigen Elektrodenleiter, was mit hohen Herstellungskosten verbunden ist.

Zudem ist die Anordnung der einzelnen Elektrodenleiter sowie der daraus gebildeten Plasmaelektroden von großer Bedeutung für die Effizienz der Plasmareinigung, so dass bereits geringfügige Abweichungen, wie sie bei einer manuellen Herstellung unvermeidbar sind, zu merklichen Unterschieden in der Reinigungswirkung der betreffenden Luftreinigungsmodule führen können. Die manuelle Herstellung der Luftreinigungsmodule wird oftmals zusätzlich dadurch erschwert, dass der zur Verfügung stehende Bauraum klein ist und die einzelnen Komponenten des Luftreinigungsmoduls unter beengten Raumverhältnissen montiert sowie anschließend in dem Strömungskanal installiert werden müssen.

Es wird deshalb als eine Aufgabe der vorliegenden Erfindung angesehen, ein Luftreinigungsmodul mit einer Plasmaerzeugungseinrichtung zur Erzeugung eines nichtthermischen Plasmas so auszugestalten, dass eine kostengünstige Montage des Luftreinigungsmoduls und dessen Einbau in einem Strömungskanal ermöglicht werden.

Diese Aufgabe wird erfindungsgemäß dadurch gelöst, dass das Kamingehäuse zylinderförmig mit einer runden oder polygonförmigen Querschnittsfläche ist und die mindestens eine Luftaustrittsöffnung in der Mantelfläche des Kamingehäuses zwischen der ersten Stirnseite und der zweiten Stirnseite angeordnet ist, und dass an der Trägerplatte mehrere zur Lufteintrittsöffnung gerichtete Elektrodenträger angeordnet sind, an denen die Elektrodenleiter derart festgelegt sind, dass die Elektrodenleiter zwischen den Elektrodenträgern verspannt sind, wobei die Elektrodenleiter längs einer die Elektrodenträger umgebenden Umfangslinie mehrfach um die Elektrodenträger gewunden sind, so dass die Elektrodenleiter eine die Elektrodenträger umgebende Mantelfläche aufspannen.

Es hat sich gezeigt, dass effiziente und einfach zu montierende Plasmaelektroden in besonders einfacher Weise kostengünstig und automatisierbar durch jeweils mindestens einen flexiblen drahtförmigen Elektrodenleiter gebildet werden können, falls die Elektrodenleiter von zwei Plasmaelektroden entlang eines Plasmaerzeugungsabschnitts miteinander verdrillt oder parallel nebeneinander angeordnet sind. Über die gesamte Länge des Plasmaerzeugungsabschnitts, in dem die beiden Elektrodenleiter eng aneinander anliegen und nur durch ein Isolierungsmaterial voneinander beabstandet sind, kann durch Anlegen einer geeigneten Wechselspannung ein längs des Plasmaerzeugungsabschnitts gebildetes nichtthermisches Plasma erzeugt werden. Als Isolierungsmaterial kann ein geeignetes dielektrisches Feststoffmaterial verwendet werden. Die Plasmaerzeugungseffizienz ist dabei wesentlich größer als bei herkömmlichen Plasmaelektroden, bei denen die drahtförmigen Elektrodenleiter gitterförmig angeordnet, bzw. miteinander verwoben sind. Die miteinander verdrillten oder nebeneinander angeordneten Elektrodenleiter können in einfacher Weise an der Trägerplatte festgelegt werden. Es können auch mehr als zwei Elektrodenleiter miteinander verdrillt oder nebeneinander angeordnet werden.

Um eine möglichst effiziente Luftreinigung zu ermöglichen ist es vorteilhaft, über einen großen Volumenbereich ein möglichst gleichmäßiges nichtthermisches Plasma zu erzeugen. Erfindungsgemäß ist zu diesem Zweck vorgesehen, dass an der Trägerplatte mehrere zur Lufteintrittsöffnung gerichtete Elektrodenträger angeordnet sind, an denen die Elektrodenleiter derart festgelegt sind, dass die Elektrodenleiter zwischen den Elektrodenträgern verspannt sind. Die Elektrodenträger können beispielsweise Stäbe oder Stangen mit einer kreisrunden, ovalen oder polygonalen Querschnittsfläche sein. Es ist ebenfalls möglich, komplex geformte Elektrodenträger zu verwenden, die eine Verlegung der Elektrodenleiter entlang von ebenen, abgekanteten oder gekrümmten Außenwandabschnitten der Elektrodenträger auf gegebenenfalls komplex ausgebildeten Verlegepfaden ermöglichen. Die Elektrodenträger können aus einem geeigneten Kunststoffmaterial, aus Keramikmaterial oder aus Metall hergestellt und gegebenenfalls mit einer Beschichtung versehen sein. Die Elektrodenträger können mit der Trägerplatte formschlüssig, über Rastmittel oder Schrauben, oder aber klebend verbunden sein. Die Elektrodenleiter können auch einstückig an der Trägerplatte ausgebildet sein.

Die von der Trägerplatte weg sich erstreckenden Elektrodenträger bilden ein Gerüst und ermöglichen es, dass zwischen den Elektrodenträgern die Elektrodenleiter geführt und verspannt werden können. Auf diese Weise können mit den Elektrodenleitern von der Trägerplatte abstehende, bzw. vorspringende flächige Gebilde hergestellt werden, die aus einer Anzahl von gradlinig verlaufenden Plasmaerzeugungsabschnitten der Elektrodenleiter bestehen. Es ist ebenfalls möglich, dass eine größere Anzahl von Elektrodenträgern an der Trägerplatte so angeordnet und ausgerichtet sind, dass die dazwischen verspannten Elektrodenleiter den Kamininnenraum so ausfüllen, dass ein sich im Wesentlichen über den gesamten Kamininnenraum erstreckendes, homogenes nichtthermisches Plasma erzeugt werden kann. Die Elektrodenleiter können an den Elektrodenträgern beispielsweise mit einem geeigneten Klebemittel oder mit Befestigungsklemmen festgelegt werden. Die derart zwischen den Elektrodenträgern verspannten Elektrodenleiter werden nicht über eine Spannkraft an den Elektrodenträgern fixiert und können auch biegeschlaff bzw. nicht gradlinig verlaufend zwischen den Elektrodenträgern angeordnet sein. Die Elektrodenträger können beispielsweise wellenförmig ausgestaltet sein oder beabstandet zueinander einzelne vorspringende Ausformungen oder Nuten aufweisen, um eine Positionierung der Elektrodenleiter an den Elektrodenträgern zu erleichtern.

Erfindungsgemäß ist vorgesehen, dass auch das Kamingehäuse zylinderförmig mit einer runden oder polygonförmigen Querschnittsfläche ist. Ein hohlzylinderförmiges Kamingehäuse, an dessen erster Stirnseite eine großflächige Lufteintrittsöffnung angeordnet ist und dessen zweite Stirnseite von der Trägerplatte verschlossen ist, eignet sich besonders vorteilhaft für die Anordnung des Luftreinigungsmoduls in einem standardisierten, bzw. üblichen Abluftkamin einer Dunstabzugshaube. Die äußeren Abmessungen des Kamingehäuses können an die Durchmesser, bzw. Querschnittsflächen der Abluftkamine, bzw. der Dunstabzugshauben Abluftkanäle angepasst sein.

Die Trägerplatte kann beispielsweise längs eines Umfangsrandes einzelne Luftaustrittsöffnungen aufweisen, die schlitzartig ausgebildet und beabstandet zueinander angeordnet sind. Durch die Anordnung der Trägerplatte an der zweiten Stirnseite des Kamingehäuses wird die durch die an der ersten Stirnseite angeordnete Lufteintrittsöffnung einströmende Luftströmung umgelenkt, bzw. verlangsamt, so dass die Verweildauer der Luftströmung in dem Bereich der Plasmaerzeugungseinrichtung erhöht und dadurch die Effizienz der Plasmareinigung der Luftströmung verbessert wird.

Es ist erfindungsgemäß vorgesehen, dass die mindestens eine Luftaustrittsöffnung in einer Mantelfläche des Kamingehäuses zwischen der ersten Stirnseite und der zweiten Stirnseite angeordnet ist. Die an der ersten Stirnseite eintretende Luftströmung wird dadurch umgelenkt und strömt seitlich aus dem Kamingehäuse aus. Durch eine geeignete Anordnung und Dimensionierung der Luftaustrittsöffnungen in der Mantelfläche des Kamingehäuses können die Umlenkung bzw. Strömungsführung der Luftströmung in dem Kamininnenraum sowie die Verweildauer, bzw. die Strömungsdrosselungseigenschaften der Luftströmung in dem Kamininnenraum so beeinflusst und angepasst werden, dass eine möglichst effiziente und hohe Beseitigung von Geruchsstoffen und flüchtigen organischen Verbindungen bei einer möglichst geringen Beeinträchtigung der durch das Luftreinigungsmodul hindurch strömenden Luftströmung erfolgen kann. Für die Ausgestaltung und Anordnung von Lufteintrittsöffnungen und von Luftaustrittsöffnungen können auch weitere Eigenschaften wie beispielsweise die Geräuschentwicklung berücksichtigt werden.

Gemäß einer besonders vorteilhaften Ausgestaltung des Erfindungsgedankens ist vorgesehen, dass an der Trägerplatte drei oder mehr vorzugsweise stabförmige Elektrodenträger angeordnet sind und dass die Elektrodenleiter längs einer die Elektrodenträger umgebenden Umfangslinie mehrfach um die Elektrodenträger gewunden sind, so dass die Elektrodenleiter eine die Elektrodenträger umgebende Mantelfläche aufspannen. Drei stabförmige Elektrodenträger können eine dreieckige Grundfläche aufspannen, so dass die um die Elektrodenträger gewickelten Elektrodenleiter ein prismenartiges Gebilde mit einer dreieckartig aufgespannten Mantelfläche bilden. Bei vier oder mehr Elektrodenträgern, deren Anordnung eine gleichmäßige oder unregelmäßige Vieleckfläche auf der Trägerplatte bildet, können die von außen um die Elektrodenträger gewundenen Elektrodenleiter eine säulenartige Mantelfläche mit einer polygonförmigen Querschnittsfläche bilden. Das Umwinden, bzw. Umwickeln der mehreren stabförmigen Elektrodenträger kann sehr schnell und zuverlässig mit Hilfe von geeigneten Wickelautomaten durchgeführt werden.

Vorzugsweise ist vorgesehen, dass die Plasmaerzeugungseinrichtung mit der Trägerplatte gesondert hergestellt und nachträglich mit dem Kamingehäuse verbunden ist. Die Plasmaerzeugungseinrichtung ist an der Trägerplatte von nahezu allen Seiten frei zugänglich, wodurch deren Herstellung erleichtert und beschleunigt werden kann. Nach der Fertigstellung der Plasmaerzeugungseinrichtung kann diese zusammen mit der Trägerplatte so an dem Kamingehäuse befestigt werden, dass die Plasmaerzeugungseinrichtung in den Kamininnenraum ragt oder vollständig in dem Kamininnenraum angeordnet ist.

Gemäß einer besonders vorteilhaften Ausgestaltung des Erfindungsgedankens ist vorgesehen, dass die Plasmaerzeugungseinrichtung mit der Trägerplatte lösbar an dem oder in dem Kamingehäuse festgelegt ist. Auf diese Weise kann die Plasmaerzeugungseinrichtung bei Bedarf von dem Kamingehäuse gelöst und zusammen mit der Trägerplatte aus dem Luftreinigungsmodul herausgenommen werden, um beispielsweise eine Reparatur vorzunehmen. Auch ein Austausch der Plasmaerzeugungseinrichtung ist in einfacher Weise sowie ohne Fachkenntnisse und gegebenenfalls ohne gesonderte Werkzeuge möglich.

Da in einer zu reinigenden Abluft regelmäßig nicht nur Geruchsmoleküle und flüchtige organische Verbindungen enthalten sind, die mit der Plasmaerzeugungseinrichtung umgewandelt und aus dem Luftstrom entfernt werden können, sondern oftmals auch kleine Partikel, Wasser- oder Fetttröpfchen und Verunreinigungen enthalten sind, ist erfindungsgemäß vorgesehen, dass die mindestens eine Luftaustrittsöffnung von einer flächigen Teilchenfiltereinrichtung bedeckt ist. Als Teilchenfiltereinrichtung können beispielsweise Vliesstoffe, textile Gelege oder ein Metallgestrick verwendet werden. Es ist ebenfalls möglich, dass die Teilchenfiltereinrichtung ein geeignetes Absorptionsmaterial, Adsorptionsmaterial oder Bindematerial für flüssige oder feste Partikel aufweist. Die Teilchenfiltereinrichtung kann beispielsweise auch Aktivkohle oder Zeolith enthalten.

Die Teilchenfiltereinrichtung kann von dem Kamininnenraum aus an die Luftaustrittsöffnungen angelegt werden und diese bedecken. Es ist ebenfalls möglich, dass das Kamingehäuse und insbesondere die Mantelfläche des Kamingehäuses doppelwandig ausgeführt ist und in einem Innenraum der Kaminwandung die Teilchenfiltereinrichtung angeordnet ist. Gegebenenfalls kann die Teilchenfiltereinrichtung entnommen und regeneriert oder erneuert werden. Es ist ebenfalls möglich, dass auch die Lufteintrittsöffnung von einer flächigen Teilchenfiltereinrichtung bedeckt ist. Dabei kann auch ein mechanischer Prallfilter oder eine Abscheideeinrichtung als Teilchenfiltereinrichtung verwendet werden.

Um die Strömungseigenschaften durch das Luftreinigungsmodul hindurch zu verbessern und gegebenenfalls den Erfassungsbereich für die eintretende Luftströmung vorgeben oder erfassen zu können ist vorgesehen, dass in Strömungsrichtung vor der Lufteintrittsöffnung eine trichterförmige Luftsammeleinrichtung angeordnet ist. Die trichterförmige Luftsammeleinrichtung kann an einem dem Kamingehäuse abgewandten Endabschnitt eine Formgebung und Abmessung aufweisen, die an standardisierte Abluftkanäle oder aber an Strömungserzeugungseinrichtungen wie beispielsweise Lüfter oder Ventilatoren angepasst ist. Auf diese Weise kann das Luftreinigungsmodul ohne zusätzliche Montagearbeiten mit einem Lüfter oder Ventilator verbunden werden. Zudem kann die trichterförmige Luftsammeleinrichtung lösbar, bzw. auswechselbar ausgebildet und mit dem Kamingehäuse verbunden sein, um ohne größeren Aufwand eine Anpassung an verschiedene Lüfter oder Abluftkanalgehäuse vornehmen zu können.

Zur Erleichterung der Montage des Luftreinigungsmoduls ist vorgesehen, dass das Kamingehäuse Anschlusseinrichtungen zur Festlegung des Kamingehäuses in einem Abluftkanal oder zur Festlegung des Kamingehäuses an einer Strömungserzeugungseinrichtung, insbesondere einer Strömungserzeugungseinrichtung einer Dunstabzugshaube aufweist. Die Anschlusseinrichtungen können beispielsweise vorspringende Befestigungslaschen oder Befestigungszungen sein. Es ist ebenfalls möglich, dass das Kamingehäuse einen seitlich vorspringenden Befestigungsflansch oder an mindestens eine der beiden Stirnseiten einen vorspringenden Befestigungskragen aufweist.

Die Erfindung betrifft auch ein Verfahren zur Herstellung einer Plasmaerzeugungseinrichtung mit mindestens zwei Plasmaelektroden. Die Plasmaelektroden werden jeweils durch mindestens einen flexiblen drahtförmigen Elektrodenleiter gebildet, wobei die Elektrodenleiter von zwei Plasmaelektroden entlang mindestens eines Plasmaerzeugungsabschnitts miteinander verdrillt oder parallel miteinander angeordnet und nur durch ein Isolierungsmaterial voneinander beabstandet sind, so dass über die gesamte Länge des Plasmaerzeugungsabschnitts ein nichtthermisches Plasma erzeugt werden kann. Erfindungsgemäß ist vorgesehen, dass die Elektrodenleiter derart an Elektrodenträgern festgelegt werden, die von einer Trägerplatte abstehen, dass die Elektrodenleiter zwischen den Elektrodenträgern verspannt sind. Beispielsweise können von einer ebenen Trägerplatte zwei senkrecht zu der Trägerplatte ausgerichtete Elektrodenträger an der Trägerplatte festgelegt werden. Die Elektrodenträger können beispielsweise aus einem geeigneten Kunststoffmaterial, aus Metall oder aus einem organischen Material wie beispielsweise Holz bestehen. Die Elektrodenträger können zudem eine geeignete Beschichtung aufweisen. Es ist ebenfalls möglich, dass die Elektrodenträger einstückig an der Trägerplatte ausgebildet werden.

Die Elektrodenträger können stabförmig ausgestaltet sein oder aber eine komplexe Formgebung mit gekrümmt oder abgekantet verlaufenden, gegebenenfalls auch unterbrochenen Außenflächen aufweisen.

Die Elektrodenleiter können beispielsweise durch Umwickeln um die Elektrodenträger herum gewunden werden, so dass die Elektrodenleiter mehrfach zwischen den beiden Elektrodenträgern verlaufen und dabei im Wesentlichen gradlinig verlaufende Plasmaerzeugungsabschnitte bilden. Die zwischen den Elektrodenträgern erzeugten Plasmaerzeugungsabschnitte können ein im Wesentlichen ebenes, flächiges Gebilde erzeugen, in dessen Umgebung das nichtthermische Plasma erzeugt wird.

Als Elektrodenleiter können geeignet isolierte Elektrodrähte oder Kabel verwendet werden. Es ist ebenfalls möglich, dass ein Elektrodenleiter ohne Isolierung mit einem zweiten Elektrodenleiter verdrillt oder verbunden wird, der eine Ummantelung aufweist, die dem zweiten Elektrodenleiter von dem ersten Elektrodenleiter trennt und isoliert.

Gemäß einer vorteilhaften Ausgestaltung des Erfindungsgedankens ist vorgesehen, dass eine Trägerplatte mindestens drei oder mehr vorzugsweise stabförmige Elektrodenträger angeordnet werden und dass die Elektrodenleiter längs einer die Elektrodenträger umgebenden Umfangslinie mehrfach um die Elektrodenträger gewunden werden, so dass die Elektrodenleiter einer die Elektrodenträger umgebende Mantelfläche aufspannen. Die von den Elektrodenleitern aufgespannte Mantelfläche ist dabei säulenförmig, wobei eine Querschnittsfläche der säulenförmigen Mantelfläche durch die Anordnung der Elektrodenträger und die von einer die Elektrodenträger umgebenden Umfangslinie gebildete Grundfläche entsprechen kann.

Nachfolgend werden Ausführungsbeispiele des Erfindungsgedankens näher erläutert, die in der Zeichnung dargestellt sind. Es zeigt:
Figur 1 eine schematische Schnittansicht eines erfindungsgemäßen Luftreinigungsmoduls,
Figur 2 eine schematische Schnittansicht eines abweichend ausgestalteten Luftreinigungsmoduls,
Figur 3 eine perspektivische Ansicht des in Figur 2 dargestellten Luftreinigungsmoduls,
Figur 4 eine perspektivische Ansicht einer Trägerplatte mit darauf angeordneten Elektrodenträgern,
Figur 5 eine perspektivische Ansicht der Trägerplatte mit einer abweichenden Anordnung von Elektrodenträgern, wobei eine um die Elektrodenträger gewundene Plasmaelektrode schematisch angedeutet ist,
Figur 6 eine schematische Draufsicht auf eine Trägerplatte, auf der sieben Elektrodenträger angeordnet und von den Plasmaelektroden umwickelt sind.
Figur 7 eine Draufsicht auf eine Trägerplatte, auf der zwei Elektrodenträger mit einer halbkreisförmigen gekrümmten Außenfläche angeordnet sind, wobei eine um die Elektrodenträger gewundene Plasmaelektrode schematisch angedeutet ist, und
Figur 8 eine perspektivische Ansicht der in Fig. 7 dargestellten Trägerplatte mit den beiden Elektrodenträgern und den schematisch dargestellten Elektrodenleitern.

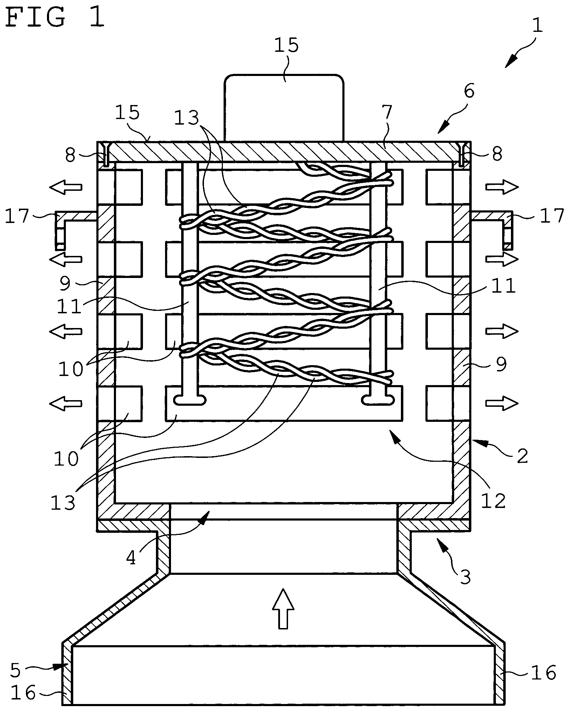

Ein in Fig. 1 exemplarisch und schematisch in einer Schnittansicht dargestelltes Luftreinigungsmodul 1 weist ein hohlzylindrisches Kamingehäuse 2 auf. An einer ersten Stirnseite 3 befindet sich eine Lufteintrittsöffnung 4. An der ersten Stirnseite 3 ist eine trichterförmige Luftsammeleinrichtung 5 angeflanscht, durch die eine Luftströmung möglichst geräuscharm und strömungsgünstig zu der verengten Querschnittsfläche der Lufteintrittsöffnung 4 geführt werden kann.

An einer der ersten Stirnseite 3 gegenüberliegenden zweiten Stirnseite 6 ist das dort offene Kamingehäuse 2 durch eine Trägerplatte 7 verschlossen. Die Trägerplatte 7 ist mit Befestigungsschrauben 8 lösbar mit dem Kamingehäuse 2 verbunden. Das Kamingehäuse 2 weist in einer rohrförmigen Mantelfläche 9 zwischen der ersten Stirnseite 3 und der zweiten Stirnseite 6 eine Anzahl von streifenförmigen Luftaustrittsöffnungen 10 auf. Die durch die erste Stirnseite 3 eintretende Luftströmung wird durch die Trägerplatte 7, die an der zweiten Stirnseite 6 angeordnet ist und als Strömungsumlenkelement dient, umgelenkt und kann durch die seitlich in der Mantelfläche 9 angeordneten Luftaustrittsöffnungen 10 wieder aus dem Kamingehäuse 2 austreten.

An der Trägerplatte 7 sind zwei stabförmige Elektrodenträger 11 so angeordnet, dass die Elektrodenträger 11 senkrecht von der Trägerplatte 7 abstehen und in einen Kamininnenraum 12 ragen. Um die Elektrodenträger 11 sind zwei miteinander verdrillte und jeweils elektrisch isolierte Elektrodenleiter 13 gewunden, so dass die beiden Elektrodenleiter 13 zwischen den Elektrodenträgern 11 verspannt sich. Die Elektrodenleiter 13 können beispielsweise mit einem geeigneten Klebemittel oder mittels Befestigungsklammern oder Spannelementen an den Elektrodenträgern 11 festgelegt werden. Es ist nicht erforderlich, dass die Elektrodenleiter 13 ausschließlich durch eine Verspannung an den Elektrodenträgern 11 gehalten und fixiert werden. Die Elektrodenträger 11 können auch vorspringende Ausformungen, Nuten oder eine wellenförmige Formgebung aufweisen, um eine Positionierung und Fixierung der Elektrodenleiter 13 an den Elektrodenträgern 11 zu erleichtern. Die beiden miteinander verdrillten Elektrodenleiter 13 bilden jeweils eine Plasmaelektrode. Durch Anlegen einer geeigneten Wechselspannung kann zwischen den beiden Elektrodenleitern 13 ein nichtthermisches Plasma erzeugt werden. Zu diesem Zweck ist an einer gegenüberliegenden Außenseite 14 der Trägerplatte 7 eine geeignete Transformatoreinrichtung 15 angeordnet, die beispielsweise an ein Spannungsversorgungsnetz eines Gebäudes angeschlossen werden kann. Durch die zwischen den beiden Elektrodenleitern 13 von der Transformatoreinrichtung 15 angelegte Wechselspannung wird längs der beiden verdrillten Elektrodenleiter 13 eine dielektrisch behinderte Entladung erzwungen und dadurch ein nichtthermisches Plasma erzeugt.

Die durch die Lufteintrittsöffnung 4 eintretende Luftströmung durchströmt den Kamininnenraum 12, wobei durch das dort gebildete nichtthermische Plasma in der Luftströmung befindliche Geruchsmoleküle und flüchtige organische Verbindungen aufgespalten und in harmlose Komponenten zerlegt werden. Die dadurch gereinigte Luftströmung tritt durch die Luftaustrittsöffnungen 10 wieder aus dem Kamingehäuse 2 aus.

Ein dem Kamingehäuse 2 abgewandter Randbereich 16 der trichterförmigen Luftsammeleinrichtung 5 weist Abmessungen und gegebenenfalls zusätzliche Befestigungselemente auf, um die trichterförmige Luftsammeleinrichtung 5 mit einer nicht dargestellten handelsüblichen Strömungserzeugungseinrichtung oder einer weiteren Filtereinrichtung verbinden zu können. Alternativ oder zusätzlich dazu können an dem Kamingehäuse 2 seitlich abstehende und abgewinkelte Befestigungslaschen 17 oder Befestigungszungen angeordnet sein, mit denen das Kamingehäuse 2 in einem Abluftkamin festgelegt werden kann. Das erfindungsgemäße Luftreinigungsmodul 1 kann dadurch in einfacher Weise und ohne nennenswerten Montageaufwand in einem Innenraum einer Dunstabzugshaube oder vorzugsweise in einem Abluftkamin einer Dunstabzugshaube angeordnet und festgelegt werden. Auch eine nachträgliche Nachrüstung bereits hergestellter oder in Betrieb befindlicher Dunstabzugshauben ist ohne größeren Aufwand möglich.

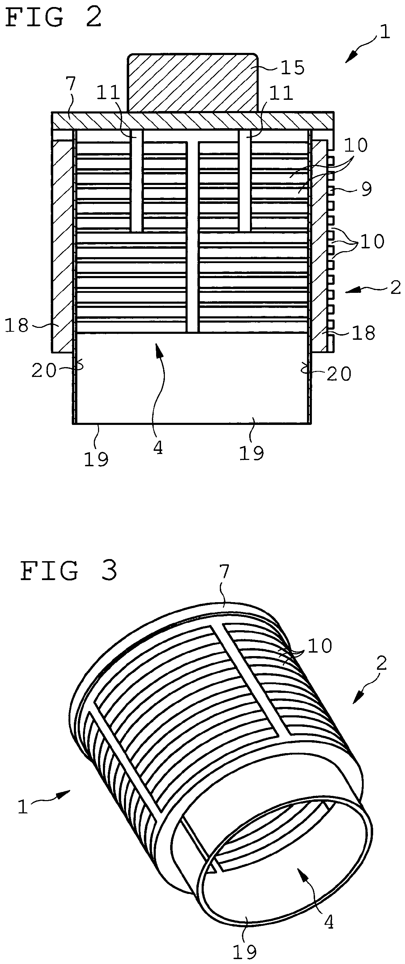

In den Fig. 2 und 3 ist eine abgewandelte Variante des erfindungsgemäßen Luftreinigungsmoduls 1 dargestellt. Das Kamingehäuse 2 weist eine doppelwandig ausgebildete Mantelfläche 9 auf. In einem Innenraum der doppelwandigen Mantelfläche 9 ist eine flächige Teilchenfiltereinrichtung 18 angeordnet. Die Teilchenfiltereinrichtung 18 kann beispielsweise ein geeignetes Vlies oder Fasergelege sein und zusätzlich Aktivkohle oder ein anderes Adsorptionsmittel enthalten. Die Teilchenfiltereinrichtung 18 kann auch ein verformbares oder formstabiles poröses Gebilde aus einem offenporigen Material, beispielsweise ein geeignetes Schaumstoffmaterial sein.

An Stelle der trichterförmigen Luftsammeleinrichtung 5 ist bei dem in den Fig. 2 und 3 gezeigten Ausführungsbeispiel ein axial vorspringender Befestigungsflansch 19 entlang eines Umfangsrands 20 der Lufteintrittsöffnung 4 angeordnet, der einstückig mit dem Kamingehäuse 2 ausgebildet oder nachträglich daran festgelegt sein kann.

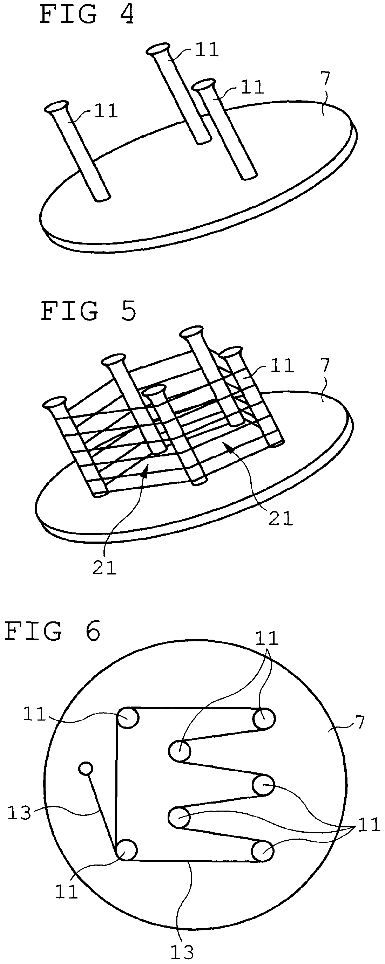

In den Fig. 4, 5 und 6 sind exemplarisch verschiedene Ausführungsbeispiele für die Anordnung von Elektrodenträgern 11 und Elektrodenleitern 13 an der Trägerplatte 7 dargestellt. Bei dem in Fig. 4 gezeigten Ausführungsbeispiel sind drei Elektrodenträger 11 so auf der Trägerplatte 7 angeordnet, dass die drei Elektrodenträger 11 eine dreieckige Grundfläche bilden. Bei dem in Fig. 5 gezeigten Ausführungsbeispiel bilden fünf Elektrodenträger 11 ein Grundfläche in Form eines gleichmäßigen Fünfecks. Die schraubenförmig von außen um die fünf Elektrodenträger 11 gewundenen und miteinander verdrillten Elektrodenleiter 13 bilden eine säulenartige Mantelfläche 21 mit einer fünfeckigen Querschnittsfläche. Bei dem in Fig. 6 gezeigten Ausführungsbeispiel sind sieben Elektrodenträger 11 so auf der Trägerplatte 7 angeordnet, dass die um die Elektrodenträger 11 gewundenen Elektrodenleiter 13 eine kammartige Querschnittsfläche aufweisen. Auf diese Weise kann in dem Kamininnenraum 12 eine große Länge der miteinander verdrillten Elektrodenleiter 13 verlegt werden und ein intensives nichtthermisches Plasma erzeugt werden, um eine möglichst hohe Reinigungseffizienz in dem zur Verfügung stehenden Kamininnenraum 12 zu ermöglichen.

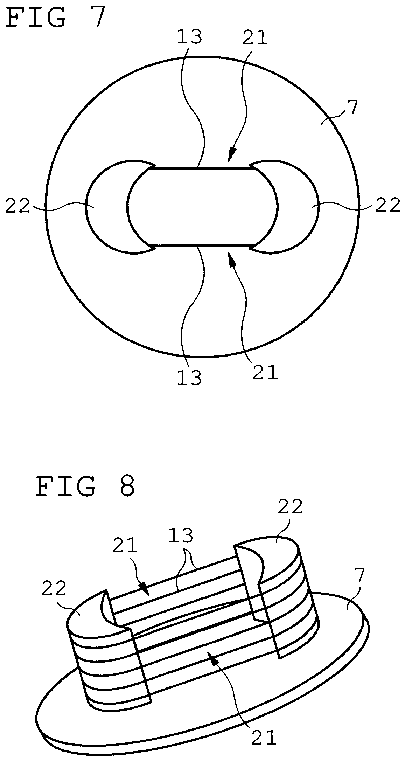

In den Fig. 7 und 8 ist exemplarisch ein weiteres Ausführungsbeispiel für die Anordnung von zwei abweichend von einer Stabform ausgestalteten Elektrodenträgern 22 auf der Trägerplatte 7 dargestellt. Die beiden Elektrodenträger 22 weisen jeweils eine halbkreisförmig gekrümmte Außenfläche auf. Durch eine Breite der Elektrodenträger 22 und den Abstand der Elektrodenträger 22 werden ein Abstand der beiden ebenen Mantelflächen 21 und deren Größe vorgegeben, in denen die um die beiden Elektrodenträgern 22 umwickelten Elektrodenleitern 13 aufgespannt sind und verlaufen. Der Abstand und die Abmessungen der Mantelflächen 21 sowie der Abstand benachbart angeordneter Abschnitte der Elektrodenleiter 13, bzw. einzelner Wicklungen der Elektrodenleiter 13 zueinander können jeweils so vorgegeben werden, dass ein möglichst homogenes und effizientes nichtthermisches Plasma in dem Kamininnenraum 12 erzeugt werden können.

## Patentansprüche

1. Luftreinigungsmodul (1) mit einem Kamingehäuse (2) zur Montage in einem Strömungskanal, wobei das Kamingehäuse (2) eine erste Stirnseite (3), eine gegenüberliegende zweite Stirnseite (6) und eine die beiden Stirnseiten (3, 6) verbindende Mantelfläche (9) aufweist und wobei an der ersten Stirnseite (3) mindestens eine Lufteintrittsöffnung (4) angeordnet ist, mit einer Trägerplatte (7) und mit einer Plasmaerzeugungseinrichtung zur Erzeugung eines nichtthermischen Plasmas, die an der Trägerplatte (7) angeordnet ist, die an der zweiten Stirnseite (6) an dem Kamingehäuse (2) so festgelegt ist, dass die Plasmaerzeugungseinrichtung in einem von dem Kamingehäuse (2) umgebenen Kamininnenraum (12) angeordnet ist, wobei die Plasmaerzeugungseinrichtung mindestens zwei Plasmaelektroden und ein Isoliermaterial aufweist, die jeweils durch mindestens einen flexiblen drahtförmigen Elektrodenleiter (13) gebildet werden, wobei die Elektrodenleiter (13) von den zwei Plasmaelektroden entlang mindestens eines Plasmaerzeugungsabschnitts miteinander verdrillt oder parallel nebeneinander angeordnet und nur durch das Isolierungsmaterial voneinander beabstandet sind, so dass über die gesamte Länge des Plasmaerzeugungsabschnitts ein nichtthermisches Plasma erzeugt werden kann, **dadurch gekennzeichnet, dass** das Kamingehäuse (2) zylinderförmig mit einer runden oder polygonförmigen Querschnittsfläche ist und die mindestens eine Luftaustrittsöffnung (10) in der Mantelfläche (9) des Kamingehäuses (2) zwischen der ersten Stirnseite (3) und der zweiten Stirnseite (6) angeordnet ist, dass die zweite Stirnseite (6) von der Trägerplatte (7) verschlossen ist, und dass an der Trägerplatte (7) mehrere zur Lufteintrittsöffnung (4) gerichtete Elektrodenträger (11) angeordnet sind, an denen die Elektrodenleiter (13) derart festgelegt sind, dass die Elektrodenleiter (13) zwischen den Elektrodenträgern (11) verspannt sind, wobei die Elektrodenleiter (13) längs einer die Elektrodenträger (11) umgebenden Umfangslinie mehrfach um die Elektrodenträger (11) gewunden sind, so dass die Elektrodenleiter (13) eine die Elektrodenträger (11) umgebende Mantelfläche (21) aufspannen.

2. Luftreinigungsmodul (1) nach Anspruch 1, **dadurch gekennzeichnet, dass** die Plasmaerzeugungseinrichtung mit der Trägerplatte (7) gesondert hergestellt und nachträglich mit dem Kamingehäuse (2) verbunden ist.

3. Luftreinigungsmodul (1) nach Anspruch 2, **dadurch gekennzeichnet, dass** die Plasmaerzeugungseinrichtung mit der Trägerplatte (7) lösbar an dem Kamingehäuse (2) festgelegt ist.

4. Luftreinigungsmodul (1) nach einem der vorangehenden Ansprüche, **dadurch gekennzeichnet, dass** die mindestens eine Luftaustrittsöffnung (10) von einer flächigen Teilchenfiltereinrichtung (18) bedeckt ist.

5. Luftreinigungsmodul (1) nach einem der vorangehenden Ansprüche, **dadurch gekennzeichnet, dass** in Strömungsrichtung vor der Lufteintrittsöffnung (4) eine trichterförmige Luftsammeleinrichtung (5) angeordnet ist.

6. Luftreinigungsmodul (1) nach einem der vorangehenden Ansprüche, **dadurch gekennzeichnet, dass** das Kamingehäuse (2) Anschlusseinrichtungen (16, 17) zur Festlegung des Kamingehäuses (2) in einem Abluftkanal oder zur Festlegung des Kamingehäuses (2) an einer Strömungserzeugungseinrichtung einer Dunstabzugshaube aufweist.

7. Verfahren zur Herstellung einer Plasmaerzeugungseinrichtung für ein Luftreinigungsmodul mit mindestens zwei Plasmaelektroden, die jeweils durch mindestens einen flexiblen drahtförmigen Elektrodenleiter (13) gebildet werden, wobei die Elektrodenleiter (13) von den zwei Plasmaelektroden entlang mindestens eines Plasmaerzeugungsabschnitts miteinander verdrillt oder parallel nebeneinander angeordnet und nur durch ein Isolierungsmaterial voneinander beabstandet sind, so dass über die gesamte Länge des Plasmaerzeugungsabschnitts ein nichtthermisches Plasma erzeugt werden kann, wobei die Elektrodenleiter (13) derart an Elektrodenträgern (11) festgelegt werden, die von einer Trägerplatte (7) abstehen, dass die Elektrodenleiter (13) zwischen den Elektrodenträgern (11) verspannt sind, **dadurch gekennzeichnet, dass** die Elektrodenleiter (13) längs einer die Elektrodenträger (11) umgebenden Umfangslinie mehrfach um die Elektrodenträger (11) gewunden werden, so dass die Elektrodenleiter (13) eine die Elektrodenträger (11) umgebende Mantelfläche (21) aufspannen.

## Claims

1. Air purification device (1) with a chimney housing (2) for mounting in a flow channel, wherein the chimney housing (2) comprises a first face side (3), an opposite second face side (6) and a lateral surface (9) that connects the two face sides (3, 6), and wherein at least one air inlet opening (4) is arranged on the first face side (3), with a support plate (7) and with a plasma generation device for the generation of a non-thermal plasma, which is arranged on the support plate (7), which is secured at the second face side (6) to the chimney housing (2) in such a manner that the plasma generation device is arranged in a chimney interior space (12) surrounded by the chimney housing (2), wherein the plasma generation device comprises at least two plasma electrodes and an insulating material, which are respectively formed by at least one flexible wire-like electrode conductor (13), wherein the electrode conductors (13) are twisted or arranged parallel next to one another from the two plasma electrodes along at least one plasma generation section and are spaced from one another only by the insulating material, so that a non-thermal plasma can be generated over the entire length of the plasma generation section, **characterized in that** the chimney housing (2) is cylindrical with a round or polygonal cross-section and the at least one air outlet opening (10) is arranged in the lateral surface (9) of the chimney housing (2) between the first face side (3) and the second face side (6), that the second face side (6) is closed by the support plate (7), and that multiple electrode carriers (11) facing towards the air inlet opening (4) are arranged on the support plate (7), on which electrode carriers the electrode conductors (13) are secured in such a way that the electrode conductors (13) are clamped between the electrode carriers (11), wherein the electrode conductors (13) are wound multiple times around the electrode carriers (11) along a circumferential line surrounding the electrode carriers (11), so that the electrode conductors (13) span a lateral surface (21) surrounding the electrode carriers (11) .

2. Air purification module (1) according to claim 1, **characterized in that** the plasma generation device is produced separately with the support plate (7), and subsequently connected with the chimney housing (2).

3. Air purification module (1) according to claim 2, **characterized in that** the plasma generation device, with the support plate (7), is releasably secured to the chimney housing (2).

4. Air purification module (1) according to any one of the preceding claims, **characterized in that** the at least one air outlet opening (10) is covered by a planar particle filter device (18).

5. Air purification module (1) according to any one of the preceding claims, **characterized in that** a funnel-like air collection device (5) is arranged in the flow direction upstream of the air inlet opening (4).

6. Air purification module (1) according to any one of the preceding claims, **characterized in that** the chimney housing (2) comprises connection elements (16, 17) to secure the chimney housing (2) in an exhaust channel, or to secure the chimney housing (2) on a flow generation device of an extractor hood.

7. Method for producing a plasma generation device for an air purification module with at least two plasma electrodes, which are respectively formed by at least one flexible wire-like electrode conductor (13), wherein the electrode conductors (13) are twisted or arranged parallel next to one another from the two plasma electrodes along at least one plasma generation section and are spaced from one another only by an insulating material, so that a non-thermal plasma can be generated over the entire length of the plasma generation section, wherein the electrode conductors (13) are secured on the electrode carriers (11), which protrude from a support plate (7), in such a manner that the electrode conductors (13) are clamped between the electrode carriers (11), **characterized in that** the electrode conductors (13) are wound multiple times around the electrode carriers (11) along a circumferential line surrounding the electrode carriers (11), so that the electrode conductors (13) span a lateral surface (21) surrounding the electrode carriers (11) .

## Revendications

1. Module de purification d'air (1) comprenant un boîtier de cheminée (2) pour le montage dans un canal de flux, dans lequel le boîtier de cheminée (2) présente une première face frontale (3), une deuxième face frontale (6) opposée et une surface latérale (9) reliant les deux faces latérales (3, 6) et dans lequel à la première face latérale (3) est disposé au moins une ouverture d'entrée d'air (4), avec une plaque de support (7) et avec un moyen de génération de plasma pour la génération d'un plasma non-thermique, qui est disposé à la plaque de support (7), et qui est fixée à la deuxième face frontale (6) au boîtier de cheminée (2) de telle manière, à ce que le moyen de génération de plasma est disposé dans un espace intérieur de cheminée (12) entouré par le boîtier de cheminée (2), dans lequel le moyen de génération de plasma présente au moins deux électrodes à plasma et un matériau isolant, qui sont formés d'au moins un conducteur d'électrodes (13) flexible sous forme de fil, dans lequel les conducteurs d'électrodes (13) à partir des deux électrodes de plasma sont disposés torsadés l'un avec de l'autre ou l'un parallèle à l'autre côte à côte le long d'au moins une partie de génération de plasma et sont seulement espacés l'un de l'autre au moyen du matériau isolant, de sorte qu'un plasma non-thermique peut être généré sur toute la longueur de la partie de génération de plasma, **caractérisé en ce que** le boîtier de cheminée (2) est cylindrique avec une surface de section transversale ronde ou polygonale et que l'au moins une ouverture de sortie d'air (10) est disposée dans la surface latérale (9) du boîtier de cheminée (2) entre la première face frontale (3) et la deuxième face frontale (6), **en ce que** la deuxième face frontale (6) est fermée par la plaque de support (7), et **en ce que** à la plaque de support (7) plusieurs supports d'électrodes (11) sont disposés orientés vers l'ouverture d'entrée d'air (4), auxquels les conducteurs d'électrodes (13) sont fixés de telle manière que, les conducteurs d'électrodes (13) sont serrés entre les supports d'électrodes (11), dans lequel les conducteurs d'électrodes (13) sont enroulés plusieurs fois autour des supports d'électrodes (11), le long d'une ligne périphérique entourant les supports d'électrodes (11), de sorte que les conducteurs d'électrodes (13) définissent une surface latérale (21) entourant les supports d'électrodes (11).

2. Module de purification d'air (1) selon la revendication 1, **caractérisé en ce que** le moyen de génération de plasma est produit séparément de la plaque de support (7) et est relié ultérieurement avec le boîtier de cheminée (2).

3. Module de purification d'air (1) selon la revendication 2, **caractérisé en ce que** le moyen de génération de plasma est fixé de façon amovible au boîtier de cheminée (2) avec la plaque de support (7).

4. Module de purification d'air (1) selon l'une quelconque des revendications précédentes, **caractérisé en ce que** l'au moins une ouverture de sortie d'air (10) est recouverte d'un moyen formant filtre à particules (18).

5. Module de purification d'air (1) selon l'une quelconque des revendications précédentes, **caractérisé en ce qu'**un moyen de collecteur d'air (5) en forme de filtre est disposé, en direction du flux, devant l'ouverture d'entrée d'air (4).

6. Module de purification d'air (1) selon l'une quelconque des revendications précédentes, **caractérisé en ce que** le boîtier de cheminée (2) présente des moyens de raccordement (16, 17) pour la fixation du boîtier de cheminée (2) dans un conduit d'air vicié ou pour la fixation du boîtier de cheminée (2) à un moyen de génération de flux d'une hotte aspirante.

7. Procédé destiné à la production d'un moyen de génération de plasma pour un module de purification d'air avec au moins deux électrodes de plasma, qui sont chacune formées par au moins un conducteur d'électrodes (13) flexible sous forme de fil, dans lequel les conducteur d'électrodes (13) à partir des deux électrodes de plasma sont disposés torsadés l'un avec de l'autre ou l'un parallèle à l'autre côte à côte le long d'au moins une partie de génération de plasma et sont seulement espacés l'un de l'autre au moyen du matériau isolant, de sorte qu'un plasma non-thermique peut être généré sur toute la longueur de la partie de génération de plasma, dans lequel les conducteurs d'électrodes (13) sont fixés aux supports d'électrodes (11) qui font saillie sur une plaque de support (7), de sorte que les conducteurs d'électrodes (13) sont serrés entre les supports d'électrodes (11), **caractérisé en ce que** les conducteurs d'électrodes (13) sont enroulés plusieurs fois autour des supports d'électrodes (11), de sorte que les conducteurs d'électrodes (13) définissent une surface latérale (21) entourant les supports d'électrodes (11).
